# EUROPEAN PATENT APPLICATION

(11) **EP 4 368 226 A1**
(43) Date of publication of application: **15.05.2024**
(21) Application number: 22927586.2
(22) Date of filing: 24.11.2022
(51) Int. Cl.: A61M 5/50, A61M 5/32, A61M 5/315

(54) **SYRINGE DEVICE FOR REUSE PREVENTION**

(30) Priority: 19.09.2022 KR 20220117894
(71) Applicant: PoongLim Pharmatech Inc, Gunsan-si, Jeollabuk-do 54001 (KR)
(72) Inventor: CHO, Hee Min, Gunsan-si, Jeollabuk-do 54153 (KR)
(74) Representative: Cabinet Chaillot
(86) International application number: PCT/KR2022/018668
(87) International publication number: WO 2024/063204

(57) **Abstract**

A disposable injection device according to the present disclosure includes a syringe barrel (10) having a cylinder-shaped internal structure; a plunger rod (20) which is coupled to the syringe barrel (10) and movably disposed to make a sliding reciprocating motion to inject a medicine contained inside; and an adaptor (40) which is coupled to an upper end portion of the syringe barrel (10) to prevent separation of the plunger rod (20) coupled to the syringe barrel (10), wherein when the plunger rod (20) moves toward a syringe needle coupled to a lower end of the syringe barrel (10), a locking flange (25) protruding in a radial direction of the plunger rod (20) is inserted into a flange locking hole (420) passing through the adaptor (40), and subsequently when the plunger rod (20) is pulled in a rearward direction, the locking flange (25) is fit into the flange locking hole (420) to restrain movements in the rearward direction, thereby preventing syringe reuse.

## Description

### [Technical Field]

The present disclosure relates to a reuse prevention injection device, and more particularly, to an approach to prevent reuse of an injection device, in which after the injection of a liquid contained in a disposable syringe is completed, a plunger rod is forcibly coupled to a syringe barrel to prevent the separation of the plunger rod.

### [Background Art]

In general, syringes are one of medical instruments, and have a wide range of uses such liquid injection, or blood or sample collection.

A syringe includes a syringe barrel containing a liquid, a syringe plunger to inject the liquid or collect blood or samples by applying pressure to the inside of the syringe barrel, a syringe needle connected to a top outlet port of the syringe barrel to inject the liquid into the skin and a syringe cap to protect the syringe needle, and the syringe needle or the syringe cap, or a blood collection tool is coupled to the top outlet port of the syringe barrel according to the use.

Syringes must be only used once, and used syringes retain blood of patients who have used the syringe needles, and in case that the syringes are reused, pathogens (hepatitis, AIDS, etc.) included in the blood are transmitted to others through the used syringes, causing secondary infection accidents, so it is necessary to dispose of used syringes.

However, often for reasons of convenience and cost savings, syringes are reused, not discarded after they have been used, and even though syringes are used many times, it is impossible to easily identify by eyes, and thus it is posed as a social issue. Several medical accidents occurred, and outbreaks of hepatitis C virus infections are typical examples.

There are main reasons of the accidents: when people with medicine addition administer medicine outside of medical institutions, they frequently reuse syringes, and some medical institutions reuse syringes due to financial reasons to reduce the cost of consumables.

As a result, there is an increasing risk of infection caused by reused syringes, and further, syringe reuse causes a social problem such as blood-borne disease infection.

Accordingly, a variety of reuse prevention techniques have been devised.

For example, Patent No. 10-1750352 discloses a syringe cap having a reuse prevention structure, including a fixed body secured to a syringe tip by fusion or welding and a separable body screw-coupled to the fixed body, and further including a reuse prevention cover which fully covers the fixed body and the separable body, wherein the reuse prevention cover includes an upper body fitted over the outer side of the separable body and a lower body fitted over the outer side of the fixed body, the upper body and the lower body are integrally connected by a connection breakable portion, the connection breakable portion is broken by an external force caused by rotation generated when rotating the upper body in one direction relative to the lower body and a pushing-up motion of the separable body from the fixed body so that the upper body is separated from the lower body and the separable body is separated from the fixed body, making it possible to identify whether the syringe has been reused by the breakage of the connection breakable portion, thereby preventing reuse of the syringe, and to easily separate the upper body of the reuse prevention cover from the lower body by the secure fixing between the fixed body and the syringe and prevent safety-related accidents caused by the connection breakable portion.

However, according to the prior art, since the separable body that is separated from the fixed body is divided into the upper body and the lower body, and when the connection breakable portion is cut or broken, the upper body and the lower body are separated from each other, it is possible to prevent reuse by separation and non-separation of the upper body and the lower body before use, and thus after the syringe is used, a syringe needle coupling portion of the syringe is exposed and a pushing action of the syringe plunger may occur, failing to completely prevent reuse of the disposable syringe.

### [RELATED LITERATURES]

Patent Literature 1: Korean Patent No. 10-1750352 (registered June 19, 2017)
Patent Literature 2: Korean Utility Model No. 20-0458761 (registered February 13, 2012)

### [Disclosure]

### [Technical Problem]

The present disclosure is directed to providing an approach to prevent reuse of an injection device, in which after the injection of a liquid contained in a disposable syringe is completed, a plunger rod is forcibly coupled to a syringe barrel to prevent the separation of the plunger rod.

After injecting the medicine in the syringe barrel through the plunger rod, the present disclosure prevents a locking flange of the plunger rod from slipping out of a flange locking hole of an adaptor after the locking flange is pushed into the flange locking hole by a downward force applied to the plunger rod in order to prevent reuse before discarding the syringe.

### [Technical Solution]

To achieve the above-described objective, a disposable injection device according to the present disclosure includes a syringe barrel 10 having a cylinder-shaped internal structure; a plunger rod 20 which is coupled to the syringe barrel 10 and movably disposed to make a sliding reciprocating motion to inject a medicine contained inside; and an adaptor 40 which is coupled to an upper end portion of the syringe barrel 10 to prevent separation of the plunger rod 20 coupled to the syringe barrel 10, wherein when the plunger rod 20 moves toward a syringe needle coupled to a lower end of the syringe barrel 10, a locking flange 25 protruding in a radial direction of the plunger rod 20 is inserted into a flange locking hole 420 passing through the adaptor 40, and subsequently when the plunger rod 20 is pulled in a rearward direction, the locking flange 25 is fit into the flange locking hole 420 to restrain movements in the rearward direction, thereby preventing syringe reuse.

The plunger rod 20 includes a rod part 21 movably coupled in upward and downward directions within the syringe barrel 10, a button part 23 disposed at an upper end of the rod part 21, and the locking flange 25 spaced apart in a downward direction of the button part 23 and protruding in the radial direction of the rod part 21.

The disposable injection device further includes a stopper 30 coupled to a lower end portion of the plunger rod 20, and the stopper 30 is coupled to a rod protrusion 27 disposed at a lower end of the rod part.

The adaptor 40 includes an adaptor plate 410 having a flange locking hole 420, and an adaptor fastening portion 430 extended in a downward direction from two sides of the adaptor plate 410 to allow coupling to a barrel connection plate 13.

The flange locking hole 420 has an upper inclined surface 423 inclined in the downward direction, a central vertical surface 424 connected to a lower end of the upper inclined surface, and a stepped portion 425 connected to a lower end of the central vertical surface 424.

The plunger rod 20 further includes a retraction prevention protrusion 29 along a periphery of the rod part 21 on an upper surface of the locking flange 25.

The locking flange 25 includes a flange bottom portion 251 horizontally extended from the rod part 21 along the radial direction, a lower inclined portion 252 extended in the upward direction outwards from an outer end of the flange bottom portion 251, an upper extended portion 253 extended in the upward direction from an outer end of the lower inclined portion 252, and a top inclined portion 254 inclined downward along the radial direction of the rod part 21 from an outer end of the upper extended portion 253.

### [Advantageous Effects]

As described above, the present disclosure may prevent reuse of the injection device by forcibly coupling the plunger rod to the syringe barrel to prevent the separation of the plunger rod after the completion of injection of the liquid contained in the medicine cartridge of the disposable syringe.

Additionally, after injecting the medicine in the syringe barrel through the plunger rod, the present disclosure may prevent the locking flange of the plunger rod from slipping out of the flange locking hole of the adaptor after the locking flange is pushed into the flange locking hole by a downward force applied to the plunger rod in order to prevent reuse before discarding the syringe.

### [Description of Drawings]

FIG. 1 is an assembled diagram of a reuse prevention injection device of the present disclosure.
FIG. 2 is an exploded diagram of the injection device of FIG. 1.
FIG. 3 is a cross-sectional view of FIG. 1, taken along the line A-A.
FIG. 4 is a detailed cross-sectional view showing a connection structure of a reuse prevention injection device of a needle according to the present disclosure.
FIG. 5 is a diagram showing a connection structure between a plunger rod and a stopper.
FIG. 6 shows an adaptor integrally formed in a syringe barrel.
FIG. 7 is an assembled diagram of another embodiment of a reuse prevention injection device according to the present disclosure.
FIG. 8 shows a plunger rod of FIG. 7 before assembly.
FIG. 9 shows a detailed structure of a plunger rod.
FIG. 10 is a cross-sectional view showing a connection structure between a syringe barrel and a plunger rod in a reuse prevention injection device.

### [Best Mode]

Hereinafter, the embodiments of the present disclosure will be described in more detail with reference to the accompanying drawings. However, the present disclosure is not limited to the following disclosed embodiments and will be embodied in a variety of different forms, and these embodiments are provided so that this disclosure will be thorough and complete, and will fully convey the scope of the present disclosure to those skilled in the art. In the drawings, the same reference sign denotes the same element.

In adding the reference signs to the elements in each drawing, it should be noted that identical elements have reference signs that are as identical as possible although they appear in different drawings. Additionally, in describing the present disclosure, when it is determined that a certain detailed description of relevant known elements or functions may obscure the subject matter of the present disclosure, its detailed description is omitted.

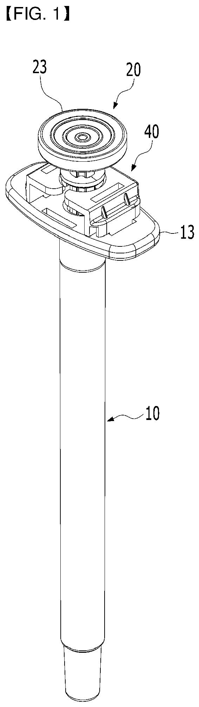

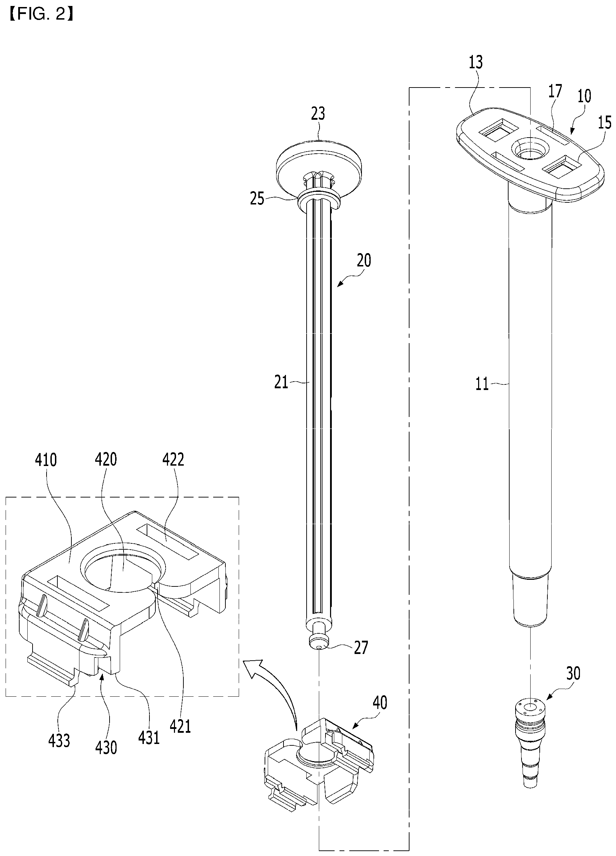

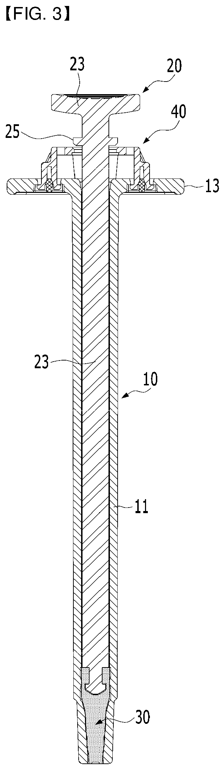

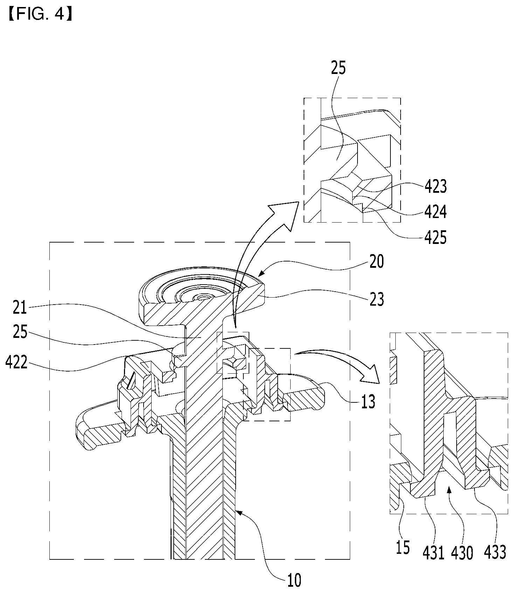

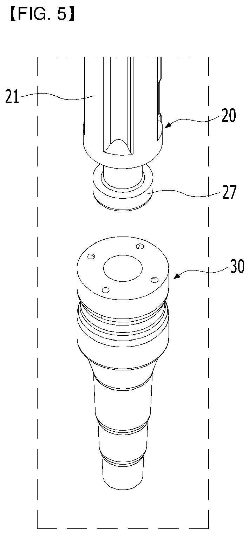

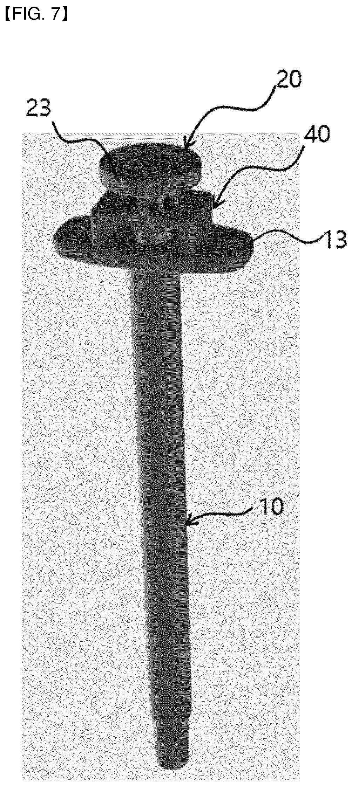

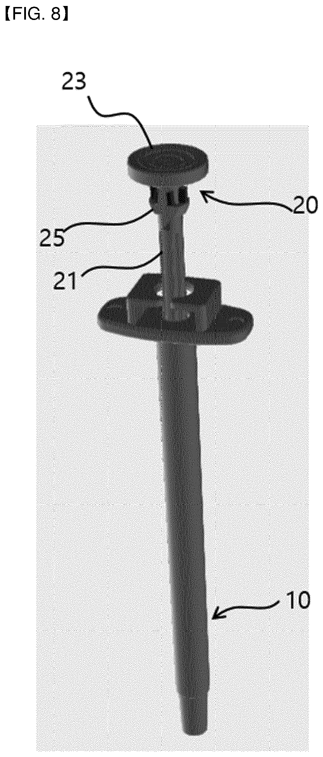

Hereinafter, an injection device having a locking structure for reuse prevention according to an embodiment of the present disclosure will be described with reference to FIGS. 1 to 10.

The reuse prevention injection device includes a syringe barrel 10 having a cylinder-shaped internal structure, a plunger rod 20 coupled to the syringe barrel 10 and movably disposed to make a sliding reciprocating motion to inject medicine contained inside, a stopper 30 coupled to the lower end portion of the plunger rod 20, and an adaptor 40 coupled to the upper end portion of the syringe barrel 10 to prevent the separation of the plunger rod 20 coupled to the syringe barrel 10.

The syringe barrel 10 includes a barrel body 11 having a hollow shape, and a barrel connection plate 13 disposed on the upper end portion of the barrel body 11 to allow the coupling of the adaptor 40. The barrel connection plate 13 has a plate shape having a generally rectangular shape, and a pair of connecting portions 15 are formed on two sides of the barrel connection plate 13 on a long axis having a longer length on the basis of the center of the barrel connection plate 13. Meanwhile, a pair of automation holes 17 are formed on two sides of the barrel connection plate 13 on a short axis having a shorter length on the basis of the center of the barrel connection plate 13. The design of the automation hole takes an automated assembly process into account, and the automation hole is configured to prevent mis-assembly that may occur when assembling the adaptor by automation.

The adaptor 40 functions to prevent the plunger rod 20 coupled to the syringe barrel 10 from moving up and down and slipping out.

The adaptor 40 includes an adaptor plate 410 having a flange locking hole 420, and an adaptor fastening portion 430 extended in the downward direction from two sides of the adaptor plate 410 to allow the coupling to the barrel connection plate 13.

The adaptor plate 410 includes the flange locking hole 420 stepped at the central part, a coupling clearance 421 that is open to one side of the flange locking hole, and a mold design hole 422 that passes through on two sides of the flange locking hole 420. The flange locking hole 420 has an upper inclined surface 423 inclined in the downward direction from the inner surface, and a central vertical surface 424 connected to the lower end of the upper inclined surface. That is, the central vertical surface 424 is extended in the vertical direction downwards from the lower end of the upper inclined surface 423 inclined in the downward direction toward the center from the upper surface of the flange locking hole 420.

After the medicine in the syringe barrel 10 is injected through the plunger rod 20, a downward force is applied to the plunger rod 20 to push a locking flange 25 of the plunger rod 20 into the flange locking hole 420 in order to prevent reuse before discarding the syringe, and the locking flange 25 is not supposed to slip out of the flange locking hole 420, so the coupling clearance 421 allows the locking flange 25 to be pushed into the flange locking hole 420.

The adaptor fastening portion 430 is coupled to the pair of connecting portions 15, and has an inner fastening portion 431 extended from the adaptor plate 410 and an outer fastening portion 433 spaced apart outside of the inner fastening portion 431. The inner fastening portion 431 and the outer fastening portion 433 have a symmetrical structure such that their lower ends protrude, facing away from each other, to allow the coupling to stepped fixed holes of the pair of connecting portions 15.

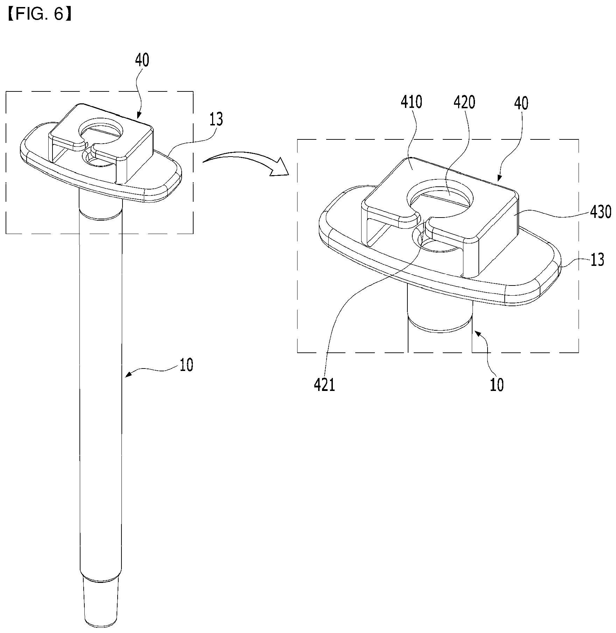

Meanwhile, referring to FIG. 6, the adaptor 40 may be integrally formed in the syringe barrel 10. That is, the adaptor 40 includes the adaptor plate 410 having the flange locking hole 420, and the adaptor fastening portion 430 extended in the downward direction from two sides of the adaptor plate 410 and integrally coupled to the barrel connection plate 13. Here, the adaptor fastening portion 430 is incorporated into the barrel connection plate 13 as a single component, but not divided into the inner fastening portion 431 and the outer fastening portion 433.

The plunger rod 20 includes a rod part 21 which is movably coupled in the upward and downward direction within the syringe barrel 10, a button part 23 disposed at the upper end of the rod part, the locking flange 25 spaced apart in the downward direction of the button part and protruding in the radial direction of the rod part, a rod protrusion 27 disposed at the lower end of the rod part and fastened to the stopper 30, and a retraction prevention protrusion 29 disposed along the periphery of the rod part 21 on the upper surface of the locking flange 25.

The locking flange 25 is coupled to the flange locking hole 420 in a mating shape, and may be a structure having a uniform thickness with the lower end inclined toward the center of the rod part.

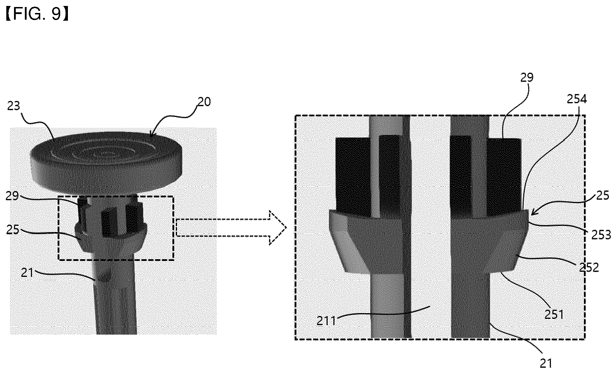

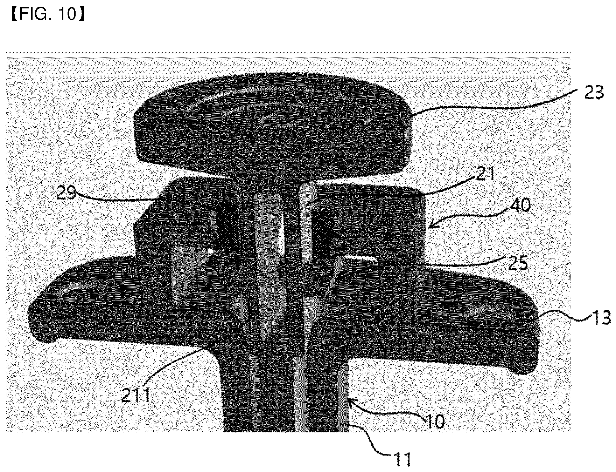

Specifically, referring to FIGS. 9 and 10, the locking flange 25 includes a flange bottom portion 251 horizontally extended from the rod part 21 along the radial direction, a lower inclined portion 252 extended in the upward direction outwards from the outer end of the flange bottom portion 251, an upper extended portion 253 extended in the upward direction from the outer end of the lower inclined portion 252, and a top inclined portion 254 inclined downward along the radial direction of the rod part 21 from the outer end of the upper extended portion 253. Through this, the top inclined portion 254 is extended along the upward direction outwards from the outer surface of the rod part 21, and meets the end of the upper extended portion 253, so the top inclined portion 254 and the upper extended portion 253 form a sharp tip.

That is, the protruding locking flange 25 of the plunger rod 20 is characterized in that when the plunger rod 20 moves toward the needle coupled to the lower end of the syringe barrel 10, the locking flange 25 is inserted into the flange locking hole 420 of the adaptor plate 410, and subsequently, when the plunger rod 20 is pulled in the rearward direction, the locking flange 25 is fit into the flange locking hole 420 to restrain movements in the rearward direction, thereby preventing reuse of the syringe.

Specifically, first, the lower inclined portion 252 of the locking flange 25 slidably moves in along the upper inclined surface 423 of the flange locking hole 420 having a similar slope angle. Subsequent to the lower inclined portion 252, the upper extended portion 253 enters the flange locking hole 420 along the upper inclined surface 423 and the central vertical surface 424, and in this process, the rod part 21 temporarily takes an elastic shrink action toward the center through a rod chamber 211 in the rod part 21. That is, when the upper extended portion 253 having a larger diameter than the inside of the central vertical surface 424 passes through the flange locking hole 420, the rod part 21 structure having the rod chamber 211 allows the flange locking hole 420 to stably enter and couple.

The retraction prevention protrusion 29 is formed along the periphery of the rod part 21, and is formed with a stepped structure more inwards than the top inclined portion 254. Through this, when the locking flange 25 is fit into the flange locking hole 420, the central vertical surface 424 of the flange locking hole 420 is surrounded through the retraction prevention protrusion 29 and the top inclined portion 254. Meanwhile, the retraction prevention protrusion 29 may be formed at a predetermined interval along the periphery of the rod part 21.

The stopper 30 coupled to the lower end portion of the plunger rod 20 is coupled through the rod protrusion of the plunger rod 20 and then coupled to the syringe barrel 10. The stopper 30 may be referred to as a plunger, a rubber stopper, a stopper or the like.

After the injection of the medicine contained in the medicine cartridge of the disposable syringe is completed through the needle, the present disclosure as described above forcibly couples the plunger rod to the syringe barrel to prevent the separation of the plunger rod, thereby preventing reuse of the injection device.

The foregoing description is made to describe the technical spirit of the present disclosure for illustrative purposes, and it is obvious to those skilled in the art that a variety of modifications and change may be made thereto without departing from the essential features of the present disclosure. Therefore, the embodiments disclosed herein are provided to describe the technical spirit of the present disclosure but not intended to be limiting, and the technical spirit and scope of the present disclosure is not limited by the embodiments. The scope of protection of the present disclosure should be interpreted by the appended claims, and it should be interpreted that the scope of protection of the present disclosure covers all the technical spirit within the equivalent scope.

### [Detailed Description of Main Elements]

10: Syringe barrel
11: Barrel body
13: Barrel connection plate
15: Connecting portion
20: Plunger rod
30: Stopper
40: Adaptor
410: Adaptor plate
420: Flange locking hole
421: Coupling clearance
422: Mold design hole
430: Adaptor fastening portion

## Claims

1. A reuse prevention injection device, comprising:
a syringe barrel (10) having a cylinder-shaped internal structure;
a plunger rod (20) which is coupled to the syringe barrel (10) and movably disposed to make a sliding reciprocating motion to inject a medicine contained inside; and
an adaptor (40) which is coupled to an upper end portion of the syringe barrel (10) to prevent separation of the plunger rod (20) coupled to the syringe barrel (10),
wherein when the plunger rod (20) moves toward a syringe needle coupled to a lower end of the syringe barrel (10), a locking flange (25) protruding in a radial direction of the plunger rod (20) is inserted into a flange locking hole (420) passing through the adaptor (40), and subsequently when the plunger rod (20) is pulled in a rearward direction, the locking flange (25) is fit into the flange locking hole (420) to restrain movements in the rearward direction, thereby preventing syringe reuse.

2. The reuse prevention injection device according to claim 1, wherein the plunger rod (20) includes a rod part (21) movably coupled in upward and downward directions within the syringe barrel (10), a button part (23) disposed at an upper end of the rod part (21), and the locking flange (25) spaced apart in a downward direction of the button part (23) and protruding in the radial direction of the rod part (21).

3. The reuse prevention injection device according to claim 1, wherein the syringe barrel (10) and the adaptor (40) are integrally formed into a single component.

4. The reuse prevention injection device according to claim 1, wherein the adaptor (40) includes an adaptor plate (410) having a flange locking hole (420), and an adaptor fastening portion (430) extended in a downward direction from two sides of the adaptor plate (410) to allow coupling to the barrel connection plate (13).

5. The reuse prevention injection device according to claim 4, wherein the flange locking hole (420) has an upper inclined surface (423) inclined in the downward direction and a central vertical surface (424) connected to a lower end of the upper inclined surface.

6. The reuse prevention injection device according to claim 2, wherein the plunger rod (20) further includes a retraction prevention protrusion (29) along a periphery of the rod part (21) on an upper surface of the locking flange (25).

7. The reuse prevention injection device according to claim 2, wherein the locking flange (25) includes a flange bottom portion (251) horizontally extended from the rod part (21) along the radial direction, a lower inclined portion (252) extended in the upward direction outwards from an outer end of the flange bottom portion (251), an upper extended portion (253) extended in the upward direction from an outer end of the lower inclined portion (252), and a top inclined portion (254) inclined downward along the radial direction of the rod part (21) from an outer end of the upper extended portion (253).
